# FASCICULE DE BREVET EUROPEEN

(11) **EP 4 451 845 B1**
(45) Date de publication et mention de la délivrance du brevet: **03.12.2025**
(21) Numéro de dépôt: 22840170.9
(22) Date de dépôt: 20.12.2022
(51) Int. Cl.: A01G 7/06

(54) **APPAREIL POUR L'INJECTION SOUS-CORTICALE D'UNE SUBSTANCE**
VORRICHTUNG ZUR SUBKORTIKALEN INJEKTION EINER SUBSTANZ
DEVICE FOR THE SUBCORTICAL INJECTION OF A SUBSTANCE

(30) Priorité: 22.12.2021 FR 2114202
(43) Date de publication de la demande: 30.10.2024
(73) Titulaire: C.E.T.E.V., 81470 Maurens-Scopont (FR)
(72) Inventeur: STEUPERAERT, Jan, 31460 Le Faget (FR); RENIER, Adeline, 31450 Pompertuzat (FR)
(74) Mandataire: Barbot, Willy
(86) Numéro de dépôt international: PCT/EP2022/086890
(87) Numéro de publication internationale: WO 2023/118071

(56) Documents cités:
- EP-A1- 3 332 630
- WO-A1-2020/208189
- FR-A1- 3 052 635
- US-A1- 2002 046 486

## Description

La présente demande de brevet revendique la priorité de la demande de brevet Français FR 2114202 déposée en date du 22/12/2022.

### Domaine Technique

La présente invention concerne le domaine de l'injection et, plus spécifiquement, un appareil destiné à l'injection sous-corticale d'une substance dans une plante.

### Technique antérieure

Dans le domaine du traitement des plantes, en particulier pour répondre à une infection par des insectes, à une infection bactérienne ou fongique, à une carence en éléments nutritifs ou pour corriger des problèmes de croissance, il est connu d'administrer une substance de type insecticide, antibiotique, antifongique, nutriment ou encore régulateur de croissance.

Une telle administration peut se faire par une application par une injection de la substance directement dans la plante, c'est-à-dire injectée sous l'écorce (c'est-à-dire de manière sous-corticale) au plus près du tissu végétal de la plante, formé de fibres et d'un système vasculaire conduisant la sève, également dénommé xylème. Le système vasculaire de la sève assure alors un transport de la substance vers les organes ciblés allant du tronc en passant par les feuilles et les éventuels fruits.

Une telle application par injection est notamment connue de la demande internationale WO2020/208189 A1 décrivant un appareil pour l'injection d'une substance sous l'écorce d'une plante comprenant un corps, une aiguille, un réservoir, des actionneurs de sorte à permettre l'insertion de l'aiguille et/ou l'injection de la substance, des capteurs d'efforts de sorte à mesurer l'effort d'appui de l'appareil contre la plante, l'effort d'insertion de l'aiguille dans l'écorce, l'effort de mise en pression du volume de substance présent dans le réservoir et un microcontrôleur de sorte à activer et moduler les actionneurs en fonctions des efforts mesurés par les capteurs.

Il à noter que des appareils plus anciens permettant une telle application par injection sont décrits dans le brevet Français FR 3052635 B1 et dans le brevet Européen EP 3 332 630 B1. Le brevet Français FR 3052635 B1 décrit un premier appareil pour l'injection sous-corticale d'une substance dans une plante qui comprend un corps, une aiguille, des actionneurs de sorte à permettre l'insertion de l'aiguille et/ou l'injection de la substance, lequel appareil comprend encore un protecteur qui entoure l'aiguille et un moyen glissière apte à permettre un mouvement d'escamotage du protecteur lorsqu'un appui est exercé sur le protecteur afin de dévoiler l'aiguille et permettre son introduction. Le brevet Européen EP 3 332 630 B1 décrit un appareil similaire à celui décrit dans le brevet Français FR 3052635 B1 et son utilisation dans un procédé d'injection sous-corticale d'une substance dans une plante avec une aiguille présentant à son extrémité une forme de « burin » avec deux biseaux opposés se rejoignant pour former un bord incisant, lequel bord incisant est positionné horizontalement lors de l'étape d'injection.

Maintenant, il s'est avéré que l'appareil décrit dans la demande internationale WO2020/208189 A1 a pu présenter des difficultés pour l'injection de substances dans des arbres dont le xylème était particulièrement dur. De surcroit, la prise en main dudit appareil s'est parfois avérée difficile pour l'utilisateur du fait de son poids et de la force d'appui à appliquer pour permettre l'insertion.

### Exposé de l'invention

Les inventeurs ont maintenant mis en évidence que l'utilisation d'un percuteur, dès lors qu'il développe une énergie seulement supérieure à 1,0 Joule, permettait l'insertion de l'aiguille sans difficultés dans tous les types d'arbres et sans avoir à apporter une force d'appui considérable comme cela pouvait être le cas avec l'appareil utilisant un vérin et sans endommager l'écorce, ni l'assise cambiale de l'arbre. Une telle valeur est relativement faible et inattendue sachant que les percuteurs dans le commerce présentent une énergie de l'ordre de 10 Joules pour les percuteurs électromécaniques et pouvant aller jusqu'à 30 Joules pour les percuteurs électropneumatiques.

En outre, les inventeurs ont développé un nouvel appareil dans lequel la tête d'injection est solidaire du corps de l'appareil et présente, avec celui-ci, un jeu de l'ordre du centimètre selon l'axe longitudinal de l'appareil correspondant à l'axe d'insertion de l'aiguille dans l'écorce. Dès lors, l'appareil se révèle particulièrement compact et d'une prise en main aisée pour l'utilisateur, tout en se révélant très efficace et fiable à l'usage.

Aussi, la présente invention vise un appareil d'injection apte à permettre l'injection d'une substance dans les écorces de plantes les plus dures tout en étant plus compact, plus robuste et plus simple d'utilisation.

A cet effet, la présente invention porte sur un appareil d'injection, avantageusement destiné à l'injection sous-corticale d'une substance dans une plante, comprenant :
▪ un corps,
▪ une tête d'injection comprenant :
   - un porte-aiguille dans lequel est maintenu une aiguille sensiblement rectiligne et définissant l'axe longitudinal de l'appareil, laquelle aiguille est percée en son centre d'un canal débouchant par au moins un trou sur la paroi latérale (c'est-à-dire la périphérie) de l'aiguille et donc pas à l'extrémité de celle-ci destinée à pénétrer dans la plante ; et
   - un guide qui présente une cavité dans laquelle le porte-aiguille et l'aiguille peuvent se mouvoir selon l'axe longitudinal de l'appareil, laquelle cavité présente une ouverture de sorte à permettre le passage de l'aiguille et son insertion dans l'écorce de la plante ;
▪ un réservoir renfermant la substance à injecter et relié au canal de l'aiguille via le porte-aiguille,
▪ un premier actionneur apte à agir sur le réservoir de sorte à mettre en pression la substance stockée dans le réservoir et à permettre l'injection de cette substance au travers de l'aiguille jusqu'à la plante via le canal,
▪ un outil d'appui de l'appareil sur la plante, lequel outil d'appui est apte à être positionné dans l'axe longitudinal de l'appareil et se trouve à proximité de l'aiguille, de préférence cet outil d'appui présente un orifice laissant passer l'aiguille,
▪ un deuxième actionneur, apte à agir sur l'outil d'appui de sorte à permettre l'extraction de l'aiguille après son insertion dans la plante.

L'appareil d'injection selon l'invention se caractérise en ce que :
1) l'outil d'appui constitue également un manchon protecteur pour l'extrémité de l'aiguille ;
2) le deuxième actionneur est également apte à rétracter l'outil d'appui au fur et à mesure de l'insertion de l'aiguille dans la plante et
3) en ce qu'il comprend également un mécanisme de frappe apte à percuter le porte-aiguille, avec une puissance inférieure à 10 Joules de sorte à permettre l'insertion de l'aiguille dans la plante.

Une telle puissance de frappe minimale est suffisante pour insérer par percussion l'aiguille dans le xylème des plantes les plus dures et, simultanément, permet de préserver l'intégrité de la plante.

Selon un mode de réalisation préféré, le porte-aiguille et l'aiguille sont solidaires du corps de l'appareil et présentent, avec celui-ci, un jeu compris entre 2 et 15 mm dans l'axe longitudinal de l'appareil, de préférence entre 3 et 10 mm et, de manière particulièrement préférée, entre 4 et 8mm, typiquement d'environ 5mm.

Les inventeurs ont en effet établi qu'un mouvement de va-et-vient du porte-aiguille et de l'aiguille sur quelques millimètres seulement dans l'axe longitudinal en association avec une faible puissance de percussion suffisait pour permettre une bonne insertion par percussion de l'aiguille dans l'écorce de la plante tout en assurant à l'appareil une compacité et une légèreté appréciable pour l'utilisateur.

Cette solidarisation de l'ensemble porte-aiguille/aiguille avec le corps de l'appareil est assurée par le guide. Maintenant, ce guide constitue également une butée qui permet de limiter le mouvement de la tête d'injection par rapport au corps selon les intervalles de jeu définis précédemment.

L'invention concerne encore un procédé de traitement d'une plante par injection sous-corticale d'une substance, lequel procédé comprend les étapes suivantes :
- application, sur l'écorce d'une plante, d'un appareil tel que décrit précédemment,
- activation de celui-ci, de sorte à permettre l'insertion de l'aiguille, au sein du xylème de la plante, puis à permettre l'injection sous-corticale de la substance, et
- extraction de l'aiguille du xylème de la plante par l'activation du deuxième actionneur dudit appareil.

L'invention concerne enfin l'utilisation d'un appareil tel que défini précédemment en vue de permettre l'injection d'une substance sous l'écorce d'une plante.

### Brève description des dessins

[Fig. 1] La figure 1 est une vue latérale en coupe d'un appareil d'injection selon l'invention ;
[Fig. 2] La figure 2 est un agrandissement de la figure 1 au niveau de la tête d'injection ; et
[Fig. 3] La figure 3 est une représentation schématique d'un mode de réalisation du porte-aiguille
[Fig. 4] La figure 4 est une représentation schématique d'un autre mode de réalisation du porte-aiguille
[Fig. 5] La figure 5 est une vue latérale en coupe au niveau de la tête d'injection en préalable à la mise en route de l'appareil ;
[Fig. 6] La figure 6 est une vue latérale en coupe au niveau de la tête d'injection au début de l'étape d'insertion;
[Fig. 7] La figure 7 est une vue latérale en coupe au niveau de la tête d'injection au milieu de l'étape d'insertion ;
[Fig. 8] La figure 8 est une vue latérale en coupe au niveau de la tête d'injection en terme de l'étape d'insertion.

### Description détaillée de l'invention

La substance à injecter peut-être de plusieurs types et appartenir à la famille des biocides (ex. insecticides, antibiotiques, antifongiques, et/ou antiparasitaires), des nutriments ou encore des régulateurs de croissance ou encore des stimulateurs de défense des plantes. Maintenant, et du fait de la destination de l'appareil selon l'invention, la substance à injecter sera plutôt une substance phytosanitaire.

Pour ce qui est du mécanisme de frappe, il présentera de préférence une puissance supérieure à 1,0 Joule, de préférence une puissance comprise entre 1,2 et 8 Joules et, de manière particulièrement préférée, entre 1,2 et 4 Joules.

Un tel mécanisme de frappe pourra être un système de percussion électromécanique ou électropneumatique.

De surcroît, ce mécanisme de frappe pourra également être un système de percussion directe ou indirecte.

A titre d'exemple, le mécanisme de frappe sera un système roto-percutant. Dans un tel système, l'aiguille et le porte aiguille n'effectueront toutefois aucune rotation dans l'axe longitudinal. En effet, et comme établi par le brevet Européen EP 3332630 B1, l'orientation du bord incisant de l'aiguille par rapport au sens des fibres de la plante est important pour l'efficacité d'injection et surtout pour limiter les dégâts à la plante lors de l'insertion de l'aiguille.

Typiquement, ce mécanisme de frappe est sous la dépendance d'un moyen d'activation, comme une gâchette, actionnable par l'utilisateur. Ce moyen d'activation permet de débuter l'insertion et il peut en outre constituer une sécurité de type homme mort. Dans ce cas, et dès lors qu'il n'est pas activé en permanence, il entraînera un arrêt de l'appareil.

Concernant l'outil d'appui, celui est avantageusement associé à un moyen de guidage dans l'axe longitudinal de l'appareil. Ce moyen de guidage assure le maintien et le centrage de l'outil d'appui le long de l'axe longitudinal de l'appareil.

De préférence, l'outil d'appui comprend un embout d'appui apte à venir en contact contre l'écorce de la plante. Cet embout d'appui peut prendre une forme plate lorsque l'écorce est lisse (ex. platane) ou une forme tridimensionnelle, notamment conique, lorsque l'écorce est crevassée (ex. pin). Un tel embout d'appui peut être réalisé dans toute matière adaptée et notamment en matière plastique, en particulier un polyamide tel que le nylon. En effet, le nylon est un matériau résistant de sorte qu'il ne s'usera pas ou faiblement au contact par frottement contre l'écorce de la plante.

Selon un mode de réalisation préféré, l'appareil d'injection comprend en outre un premier capteur d'effort apte à mesurer l'effort d'appui de l'outil d'appui contre l'écorce de la plante et au moins un microcontrôleur qui permet :
1) dès lors qu'un effort minimum, correspondant à la valeur d'activation, est détecté, l'activation du deuxième actionneur de sorte à rétracter l'outil d'appui et à permettre l'insertion de l'aiguille dans l'écorce de la plante. Avantageusement, cette valeur d'activation est d'au moins 2 daN. De préférence, l'activation du deuxième actionneur permet de rétracter l'outil d'appui à une vitesse d'au moins 10 mm/seconde, typiquement de l'ordre de 20 mm/s.
2) dès lors que l'effort d'appui est inférieur à une valeur seuil dite valeur de frappe, l'activation du mécanisme de frappe. Une telle baisse de l'effort d'appui est consécutive de l'activation du deuxième actionneur qui ne serait pas associée simultanément à l'insertion de l'aiguille dans l'arbre. En effet, ce décalage entraine alors un éloignement de l'outil d'appui de la surface de l'écorce, lequel éloignement augmente le risque que l'aiguille se torde. Avantageusement, cette valeur de frappe est inférieure à 250 N, de préférence elle est de 150 N. Avantageusement encore, le microcontrôleur diminue la vitesse du deuxième actionneur de sorte à réduire la vitesse de rétractation de l'outil d'appui. Typiquement, cette réduction de la vitesse de rétractation de l'outil d'appui est d'au moins un facteur 2, de préférence d'un facteur 4.

Pour ce qui est du mécanisme de frappe, il présentera de préférence une puissance comprise entre 1,2 et 4 Joules et, de manière particulièrement préférée entre 1,2 et 2 Joules.

Avantageusement, le microcontrôleur permet :
3) dès lors que l'effort d'appui devient inférieur à une valeur limite, une désactivation du deuxième actionneur. Une telle désactivation contribue à limiter les risques de détérioration de l'aiguille. De préférence, cette valeur limite est inférieure à 50 N. Avantageusement, le microcontrôleur augmente la puissance du mécanisme de frappe. Typiquement, cette augmentation de la puissance du mécanisme de frappe est supérieure ou égale à 50%, de préférence supérieure ou égale à 100%.

Pour ce qui est du mécanisme de frappe, il présentera de préférence une puissance comprise entre 2 et 8 Joules et, de manière particulièrement préférée entre 2 et 4 Joules.

Avantageusement encore, le microcontrôleur permet :
4) dès lors que l'effort d'appui est inférieur à la valeur limite pendant plus de 5 secondes, de préférence pendant plus de 2 secondes, une désactivation du mécanisme de frappe.

Le couple formé par ce premier capteur d'effort et le microcontrôleur constitue des éléments essentiels pour l'insertion optimale de l'aiguille dans le xylème de la plante et donc du fonctionnement de l'appareil.

Selon un mode de réalisation préféré, l'appareil d'injection comprend en outre un premier capteur de position, associé au deuxième actionneur, apte à mesurer la profondeur d'insertion de l'aiguille dans l'écorce de la plante.

Avantageusement, le microcontrôleur, en lien avec les informations transmises par ce premier capteur de position, permet :
1) dès lors que la profondeur cible est atteinte, pour la plante dans laquelle l'aiguille est insérée, une désactivation du deuxième actionneur et du mécanisme de frappe. Avantageusement, le microcontrôleur permet alors l'activation du premier actionneur, apte à agir sur le réservoir de sorte à mettre en pression la substance stockée dans celui-ci, et l'injection de la substance au travers de l'aiguille jusqu'à la plante.

A titre d'exemple, une telle profondeur cible est de 45mm pour un grand arbre tel le marronnier, le châtaignier ou le pin, 38mm pour un arbre fruitier tel le pommier, ou encore 25mm pour un arbre fin et dur comme le cerisier.

Avantageusement, le microcontrôleur permet, suite à la désactivation du mécanisme de frappe du fait d'un effort d'appui inférieur à la valeur limite pendant plus de 5 secondes, de préférence pendant plus de 2 secondes :
2) dès lors qu'une profondeur limite est atteinte, pour la plante dans laquelle l'aiguille est insérée, une activation du premier actionneur, apte à agir sur le réservoir de sorte à mettre en pression la substance stockée dans celui-ci, et l'injection de la substance au travers de l'aiguille jusqu'à la plante.
3) dès lors qu'une profondeur limite n'est pas atteinte, pour la plante dans laquelle l'aiguille est insérée, d'informer l'utilisateur que la séquence d'insertion a échoué. Une telle information peut être faite au moyen d'un témoin lumineux et/ou sonore.

En effet, en deçà d'une profondeur limite, l'injection de substance risquerait d'abimer l'assise cambiale du fait du décollement de l'écorce induit par la pression d'injection de la substance. A titre d'exemple, une telle profondeur limite est de 33mm pour un grand arbre tel le marronnier, le châtaignier ou le pin, 29mm pour un arbre fruitier tel le pommier, ou encore 18mm pour un arbre fin et dur comme le cerisier.

Le microcontrôleur peut alors activer le deuxième actionneur pour extraire l'aiguille de l'écorce de l'arbre, en vue d'initier une nouvelle séquence d'insertion/injection à un autre emplacement de l'écorce.

En ce qui concerne l'aiguille, celle-ci présentera un diamètre compris entre 1 et 3 mm, de préférence entre 1,5 et 2 mm. En effet, un faible diamètre favorise une cicatrisation de la plante suite à la lésion créée par le passage de l'aiguille dans l'écorce de la plante lors de l'injection.

Pour ce qui est de la longueur de l'aiguille, elle est comprise entre 2 et 20 cm, de préférence entre 2 et 10 cm et, de manière particulièrement préférée entre 3 et 7 cm. Pour ce qui est de sa forme, elle pourra présenter une forme cylindrique.

Pour ce qui est du au moins un trou présent sur la paroi latérale de l'aiguille et donc pas à l'extrémité de celle-ci destinée à pénétrer dans la plante, celui-ci pourra se positionner à moins d'un centimètre de cette extrémité, voir à moins de 5 millimètres de celle-ci, notamment moins de 4 voir moins de 3 mm.

Maintenant, l'aiguille pourra comprendre plusieurs trous distaux qui forment autant de sorties du canal et qui sont aptes à permettre la dispersion de la substance dans la plante.

L'aiguille est idéalement maintenue en position, notamment grâce à l'outil d'appui décrit ci-après, et résiste bien lors de son insertion par percussion dans l'écorce de la plante.

Pour faciliter la mise en place de l'aiguille dans le porte-aiguille et son remplacement, le porte-aiguille intègre une monture. Indistinctement, cette monture peut soit être partie intégrante du porte-aiguille (c'est-à-dire ne former qu'une seule pièce avec lui), soit consister en une pièce indépendante que l'on vient fixer sur le porte-aiguille. Dans ce dernier cas, la monture d'aiguille et le porte-aiguille sont associés entre eux de manière étanche (au besoin avec rajout d'un joint) et par une fixation réversible (ex. vissage, douille ou toute forme d'attache rapide).

Dans le cas d'une monture faisant partie intégrante du porte aiguille, l'aiguille est associée au porte aiguille de façon réversible (ex. vis de blocage, circlips, vissage, etc.). Dans le cas maintenant où la monture peut être séparée du porte-aiguille, l'aiguille peut être associée à la monture de façon réversible ou non (ex. collage, soudage, brasage, etc.).

L'association de l'aiguille et de la monture se fait sur une longueur qui pourra être comprise entre 5mm et 35mm, notamment entre 15mm et 25mm, plus particulièrement sur une longueur de 20mm.

En lien maintenant avec le guide qui fait office de butée, celui-ci est peut-être associé à un moyen de rappel qui prend typiquement la forme d'un ressort. Ce moyen de rappel permet, après que le porte-aiguille ait avancé selon l'axe longitudinal de l'appareil après avoir été percuté par le mécanisme de frappe, de le ramener de nouveau vers le mécanisme de frappe pour une nouvelle percussion. Maintenant, les inventeurs ont montré qu'un tel moyen de rappel n'est pas nécessaire et que l'effort d'appui contre l'arbre lors de l'insertion de l'aiguille suffit à ramener le porte-aiguille rapidement contre le mécanisme de frappe.

Avantageusement, ce guide qui fait office de butée est amovible.

De préférence, ce guide est monté pivotant, par rapport au corps, autour d'un axe transversal perpendiculaire à l'axe longitudinal de l'appareil. De la sorte, l'utilisateur peut procéder au remplacement d'une aiguille et/ou du porte-aiguille de manière plus simple et plus rapide.

De préférence, le pivotement du guide permet également d'accéder au réservoir de substance à injecter. Il est alors possible de remplir de nouveau ce réservoir ou de le changer dans le cas où celui-ci est avantageusement monté mobile.

Concernant le réservoir, celui-ci est relié au porte-aiguille, et donc à l'aiguille et à son canal, via un conduit qui prend avantageusement la forme d'une durite.

Le réservoir prend de préférence la forme d'un contenant mobile, notamment d'une seringue.

En lien avec le premier actionneur apte à agir sur le réservoir de sorte à mettre en pression la substance stockée dans celui-ci et à permettre l'injection de cette substance au travers de l'aiguille jusqu'à la plante via le canal, il prendra idéalement la forme d'un vérin.

Selon un mode de réalisation préféré, l'appareil d'injection comprend en outre un deuxième capteur d'effort apte à mesurer l'effort de mise en pression de la substance stockée dans le réservoir, et au moins un microcontrôleur qui permet :
1) de moduler la compression du volume de substance présent dans le réservoir par le premier actionneur, et donc l'injection de cette substance dans la plante, en fonction de l'effort mesuré par le deuxième capteur d'effort.
2) dès lors que l'effort de mise en pression est trop important, d'inactiver le premier actionneur de sorte à interrompre l'injection. Un tel effort de pression trop important correspond à un effort supérieur ou égale à 100 daN, de préférence supérieure ou égale à 200 daN. Un tel effort de mise en pression trop important peut être le fait, par exemple, d'une zone trop dure dans la plante ou d'une obturation du trou de l'aiguille. Il y a alors un risque d'endommager l'arbre par décollement de l'assise cambiale, ce qui causerait un dommage irréversible sur une grande surface et surtout inacceptable dans le cadre d'un traitement, et éventuellement le réservoir, voir même l'appareil.
3) dès du fait l'effort de mise en pression est trop faible, d'inactiver le premier actionneur de sorte à interrompre l'injection. Un tel effort de pression trop faible correspond à un effort inférieur ou égal à 5 daN. Un tel effort de mise en pression trop faible peut résulter, par exemple, d'un endommagement interne de la plante. Il y a alors un risque de réaliser une injection inefficace et, surtout, de disperser inutilement des produits phytosanitaires dans l'environnement extérieur.

Dans ces deux derniers cas d'une interruption de la séquence d'injection, le microcontrôleur peut en outre informer l'utilisateur de cette interruption. Une telle information peut être faite au moyen d'un témoin lumineux et/ou sonore. Le microcontrôleur peut alors activer le deuxième actionneur pour extraire l'aiguille du xylème de l'arbre, en vue d'initier une nouvelle séquence d'insertion/injection à un autre emplacement de l'écorce.

L'appareil selon l'invention peut également comprendre un deuxième capteur de position qui est associé au premier actionneur et qui permet de renseigner l'utilisateur, et potentiellement l'appareil, sur le volume de substance introduit dans la plante. Sur la base des informations fournies par ce deuxième capteur de position, le microcontrôleur est à même d'interrompre la phase d'injection, dès lors que le volume de substance souhaité a été injecté dans la plante, ou d'enregistrer le volume de substance introduit réellement dans le cas où l'injection a été interrompue pour une des raisons précitées.

En fonction de la plante à traiter, laquelle est renseignée par l'utilisateur par exemple, il est ainsi possible de moduler le volume de substance injecté.

L'appareil est alimenté par une source de puissance qui prend la forme d'un branchement vers le secteur ou d'une batterie.

L'appareil peut éventuellement comprendre une mémoire contenant les paramètres de fonctionnement de l'appareil et les paramètres à utiliser en fonction de la plante à injecter.

Idéalement, l'appareil selon l'invention comprend un témoin de fonctionnement, comme un voyant lumineux (LED).

Avantageusement, l'appareil selon l'invention comprend des moyens de préhension, comme des poignées pour faciliter la prise en main de l'appareil par l'utilisateur.

L'appareil selon l'invention peut comprendre en outre des moyens de portage, comme des sangles pour soulager l'utilisateur du poids de l'appareil.

L'appareil comprend avantageusement une interface visuelle, comme un écran de contrôle, éventuellement tactile, par lequel l'utilisateur peut sélectionner les paramètres de contrôles (puissance de frappe, vitesse de rotation, mesures d'efforts, intensité moteur actionneur, etc.). L'interface visuelle avertit également l'utilisateur de la charge de batterie, du volume de substance dans le réservoir, etc. L'interface visuelle prévient également l'utilisateur de toute anomalie de fonctionnement de l'appareil (effort excessif détecté, fuite au niveau de la durite, etc.).

L'appareil comprend des moyens de protection du corps extérieur, comme des pads de protection protégeant l'appareil en cas de chute.

L'appareil comprend des connecteurs usuels type USB et autres pour le transfert de données. Ce transfert peut être réalisé via une connexion sans fil type WIFI ou BLUETOOTH.

En ce qui concerne le procédé de traitement d'une plante par injection utilisant l'appareil décrit précédemment, l'étape d'insertion est effectuée préférentiellement avec une puissance du mécanisme de frappe supérieure à 1,0 Joule, typiquement une puissance comprise entre 1,2 et 8 Joules et, de manière particulièrement préférée, entre 1,2 et 4 Joules.

Concernant enfin l'utilisation d'un appareil tel que décrit précédemment en vue de permettre l'injection sous-corticale d'une substance, la puissance du mécanisme de frappe est préférentiellement supérieure à 1,0 Joule, typiquement comprise entre 1,2 et 8 Joules et, de manière particulièrement préférée, entre 1,2 et 4 Joules.

L'invention sera mieux comprise à la lecture de la description qui va suivre, donnée uniquement à titre d'exemple non limitatif et faite en référence aux figures annexées sur lesquelles :

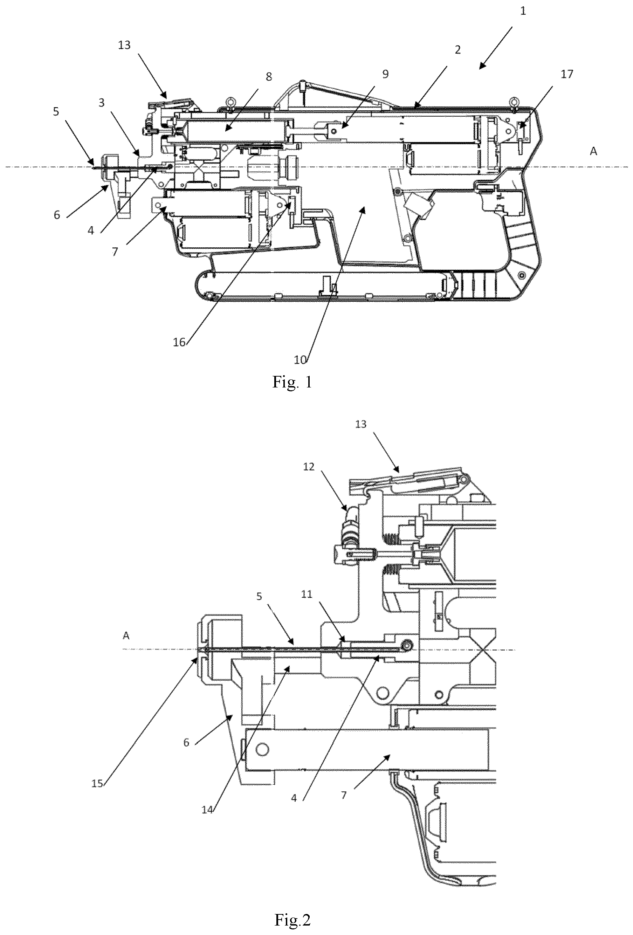

Sur la figure 1, l'appareil d'injection 1 est tenu à l'horizontal et pointe vers sa cible. L'avant de l'appareil d'injection 1 correspond à la tête d'injection, l'arrière de l'appareil permet à l'utilisateur de le prendre en main, notamment pour appliquer un effort d'appui sur l'écorce d'une plante en préalable à l'injection.

En référence à la figure 1, l'appareil d'injection 1 comprend un corps 2, également dénommé châssis ou carcasse, qui correspond à une partie usinée et qui sert de support aux diverses pièces constitutives de cet appareil. Ce support peut être ajouré à différents endroits de sorte à être le plus léger possible.

A l'extrémité avant de l'appareil d'injection 1, un système de verrouillage/déverrouillage 13, par exemple une grenouillère équipée d'un crochet, vient s'accrocher à un réceptacle correspondant à la butée 3 de la tête d'injection et ceci afin de fermer ou sécuriser l'installation de l'ensemble porte-aiguille 4 / aiguille 5.

Cette butée 3 se présente sous la forme d'un cylindre faisant saillie et dans lequel est usiné une cavité qui est une contreforme du porte-aiguille 4. Au centre de ce cylindre est également usiné un perçage qui communique avec la cavité et qui constitue une ouverture par laquelle une aiguille 5 peut passer.

L'aiguille 5 définie un axe longitudinal A de l'appareil d'injection 1.

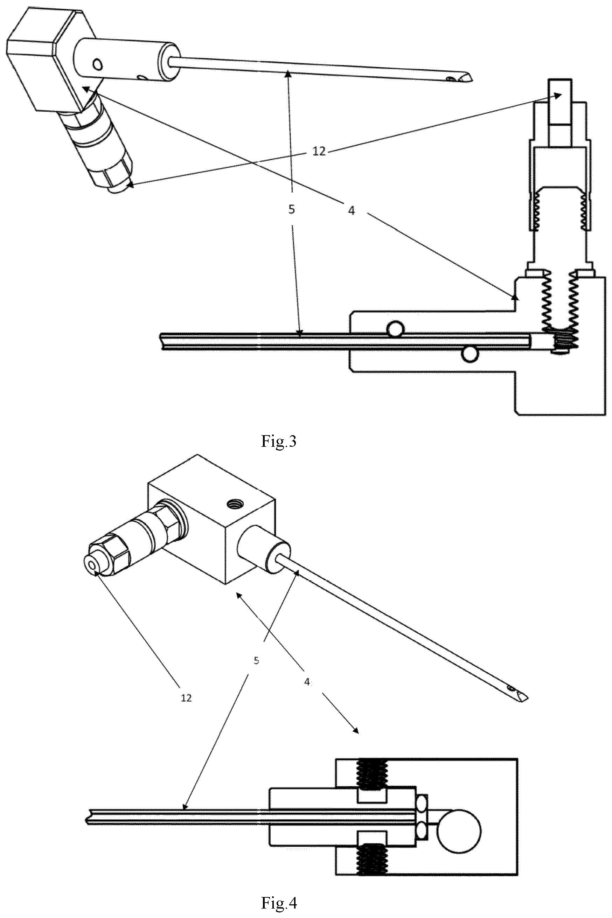

Dans la contreforme, et lorsque la butée 3 est fixée ou sécurisée sur le corps 2 de l'appareil d'injection 1, une structure possible de porte-aiguille 4 est représenté à la figure 3. Dans ce porte-aiguille, la monture d'aiguille fait partie intégrante du porte-aiguille et se présente sous la forme d'un cylindre creux, par exemple d'une longueur de 20 mm et d'un diamètre de 5mm, dans lequel est usiné un perçage qui constitue le logement de l'aiguille 5. Le canal de l'aiguille 5, une fois l'aiguille 5 mise en place dans la monture, débouche dans un autre canal usiné au sein du porte-aiguille 4, lequel autre canal est connecté, via la durite 12, au réservoir 8 de l'appareil 1. Cet autre canal comprend en outre un moyen d'immobilisation de l'aiguille 5, lequel prend la forme d'une vis qui vient en butée sur l'aiguille 5, laquelle vis est percée en son centre de sorte à laisser passer la substance de la durite 12 vers le canal de l'aiguille 5.

Une autre structure possible de porte-aiguille 4 est représentée à la figure 4. Dans cette autre porte-aiguille 4, la monture de l'aiguille 5 correspondant toujours à un cylindre creux dans lequel est usiné un perçage dans lequel l'aiguille 5 est immobilisée (ex. par collage). Maintenant, cette monture vient se loger, avec un joint torique en interface pour l'inétanchéité, dans une forme complémentaire dans le corps du porte-aiguille 4 de sorte à permettre l'assemblage du porte-aiguille 4. Le verrouillage de la monture dans le porte-aiguille 5 est assurée par une vis sans tête. Le canal de l'aiguille 5, une fois l'ensemble aiguille 5/ monture du porte-aiguille mis en place, débouche dans un autre canal usiné au sein du porte-aiguille 4, lequel autre canal est connecté, via la durite 12, au réservoir 8 de l'appareil 1.

Le guide 3 de la tête d'injection est également monté pivotant par rapport au corps 2 de l'appareil d'injection 1 autour d'un axe transversal B perpendiculaire à l'axe longitudinal A. Ainsi, lorsque l'utilisateur déverrouille le système de fixation 13 du guide 3, le guide 3 bascule par gravité autour de l'axe transversal B. Un contact de détection du guide 3 sur le corps 2 est rompu sécurisant ainsi l'appareil d'injection 1.

L'utilisateur est alors en mesure de remplir le réservoir 8, positionné au-dessus de l'appareil 1 et le long de l'axe longitudinal A, de substance ou, plus simplement de le changer, lorsque ce réservoir 8 est amovible.

Une fois le guide 3 remis en place sur l'appareil 1, le système de fixation verrouillé, la connexion entre la durite 12 et le réservoir 8, se fait à travers la partie supérieure du guide 3 par un raccord usiné au sein du guide 3 qui permet une liaison étanche entre la sortie du réservoir 8 et l'entrée de la durite 12.

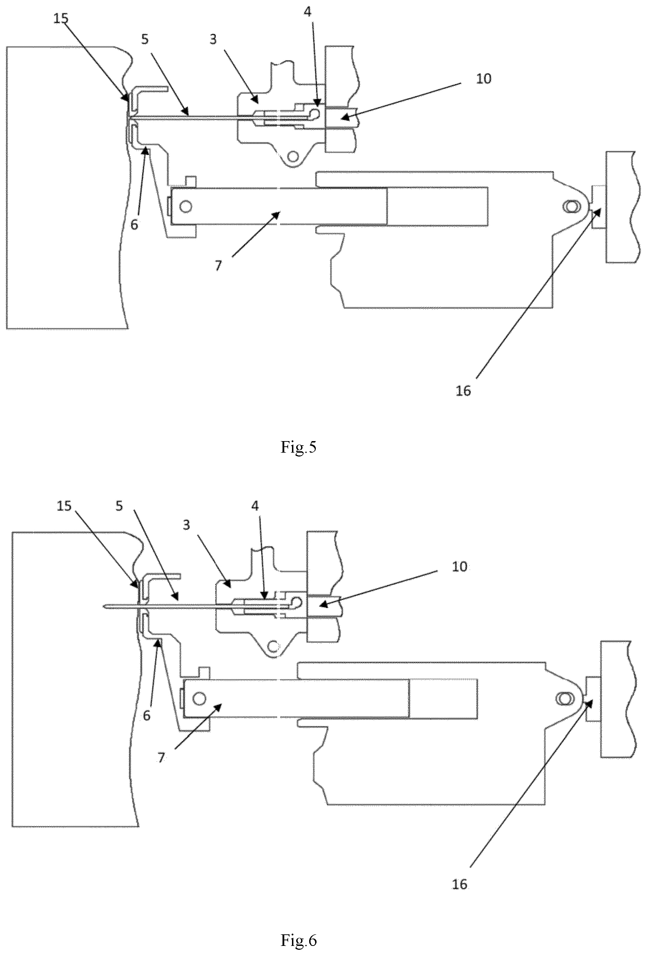

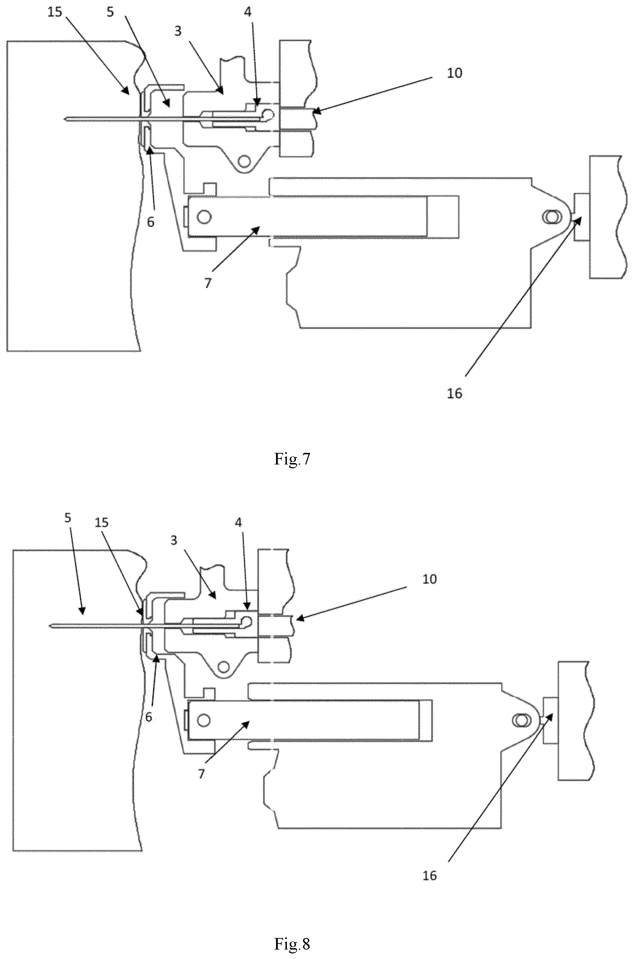

Comme cela est illustré aux figures 5 à 8, l'utilisateur vient, dans un premier temps, appliquer l'outil d'appui 6, et plus spécifiquement un embout d'appui 15, positionné dans l'axe longitudinal A, sur l'écorce de la plante. Cet embout d'appui 15 présente, sous une forme adaptée au type d'écorce, notamment plate pour les écorces lisses. L'embout d'appui 15 peut aussi être usiné d'un perçage pour le passage de l'aiguille 5. L'outil d'appui 6 est associé à un capteur d'effort 16 qui mesure l'effort d'appui de l'outil d'appui 6 contre l'écorce de la plante.

Dès lors qu'un effort minimum, correspondant à la valeur d'activation, est détecté par le premier capteur d'effort 16, le microcontrôleur active le deuxième actionneur 7 de sorte à rétracter l'outil d'appui 6 (vitesse de 20 mm/s) et à permettre l'insertion de l'aiguille 5 dans l'écorce de la plante.

L'aiguille 5 s'enfonce alors progressivement dans l'écorce de la plante. Maintenant, si l'aiguille 5 s'enfonce dans la plante moins vite que le deuxième actionneur 7 ne rétracte l'outil d'appui 6, il s'ensuit une baisse de l'effort d'appui identifiée par le premier capteur d'effort 16.

Lorsque l'effort d'appui est inférieur à une valeur seuil dite valeur de frappe, typiquement de 150 N, le microcontrôleur active le mécanisme de frappe 10 (détail du mécanisme non représenté sur la figure 1) vient percuter le porte-aiguille 4 dans l'axe longitudinal A de l'appareil 1, lui imprimant un mouvement de va-et-vient au sein du guide 3 qui permet l'insertion de l'aiguille 5 dans l'écorce de la plante.

Lors de ce mouvement de va-et-vient, l'effort d'appui ramène en arrière le porte-aiguille 4 vers le mécanisme de frappe 10. Cette frappe du mécanisme de frappe 10 est générée par une transmission mécanique ou pneumatique. Un marteau est propulsé par un piston dont le mouvement de va-et-vient est cinématiquement contrôlé ou par de l'air comprimé.

Dans ce mécanisme de frappe 10, le marteau vient donc percuter un burin, en translation libre dans son logement, qui percute ensuite le porte-aiguille 4 dans l'axe longitudinal A. Ce mécanisme est un système de percussion indirecte.

En variante, le burin n'est plus en translation libre dans son logement mais au contact du porte-aiguille 4. L'énergie de frappe générée par le marteau au contact du burin est alors transférée de manière optimale au porte-aiguille 4. C'est un système de percussion indirecte plus performant.

Lorsque le mécanisme de frappe 10 est un système de percussion directe, le marteau percute directement le porte-aiguille 4 dans l'axe longitudinal A. L'énergie de frappe générée par le marteau au contact du porte-aiguille 4 est plus importante.

Le système de percussion directe est donc plus performant que le système de percussion indirecte.

Dans ces systèmes de percussion, l'énergie de frappe générée par le marteau au contact du burin ou du porte-aiguille 4, est transférée à l'écorce de la plante lors de l'insertion par percussion de l'aiguille 5 dans celle-ci.

Lors des différents essais réalisés, on a utilisé généralement une puissance du mécanisme de frappe 10 comprise entre 1,2 et 2 Joules. Simultanément, le microcontrôleur diminue la vitesse du deuxième actionneur 7 (typiquement 5 mm/s) de sorte à réduire la vitesse de rétractation de l'outil d'appui 6 et limiter les risques de tordre l'aiguille 5.

Si l'effort d'appui redevient supérieur à la valeur de frappe, le microcontrôleur désactive le mécanisme de frappe et ramène la vitesse de rétraction de l'outil 6 à sa vitesse initiale par le biais du deuxième actionneur 7. Maintenant, et dans certaines situations, l'effort d'appui, après avoir été inférieur à la valeur de frappe a continué à diminuer, et ceci malgré l'activation du mécanisme de frappe 10. Dès lors, il a été défini une valeur limite (généralement de 50 N), en dessous de laquelle le microcontrôleur désactive le deuxième actionneur 7 et, simultanément, augmente la puissance du mécanisme de frappe 10 (généralement entre 2 et 4 Joules).

Cette séquence permet d'améliorer l'insertion de l'aiguille 5 et est arrêtée dès lors que l'effort d'appui repasse au-dessus de cette valeur limite.

Une fois l'insertion de l'aiguille 5 jusqu'à une profondeur cible dans le xylème de la plante, laquelle profondeur cible est identifiée par un premier capteur de position, le microcontrôleur peut initier l'injection de la substance.

Pour se faire, l'actionneur 9 agit sur le réservoir 8, sous le contrôle du capteur d'effort 17 à celui-ci pour mesurer l'effort de mise en pression de la substance à injecter dans le xylème de la plante. La substance passe alors du réservoir 8, entre dans la durite 12 jusqu'à l'aiguille 5 en passant par le porte-aiguille 4, avant d'être injectée dans la plante.

Une fois le volume de substance nécessaire injecté, tel que déterminé par le deuxième capteur de position associé au premier actionneur 9, l'actionneur 7 agit sur l'outil d'appui 6 pour faire avancer l'outil d'appui 6 contre l'écorce de la plante dans l'axe longitudinal A jusqu'à ce que l'aiguille 5 sorte complètement de l'écorce de la plante.

A cette fin, l'outil d'appui 6 comporte, en partie inférieure et le long de l'axe longitudinal A, un logement destiné à accueillir la tige de l'actionneur 7. De préférence, le logement de la partie inférieure de l'outil d'appui 6 est aimanté de sorte que la tige de l'actionneur 7 est en liaison magnétique non permanente avec l'outil d'appui 6. Une telle configuration permet de désengager aisément et sans outil, la tige de l'actionneur 7 et l'outil d'appui 6.

Durant cette étape d'extraction, un moyen de guidage 14, par exemple des glissières, accompagne l'outil d'appui 6 le long de l'axe longitudinal A de l'appareil 1. Ainsi, deux glissières chacune disposée de part et d'autre de la partie inférieure de la tête d'injection, le long et parallèlement à l'axe longitudinal A, coulissent dans des rails fixés à l'intérieur du corps 2 de l'appareil d'injection 1 grâce à des galets ou à des billes.

Durant cette même étape d'extraction, on peut imaginer des moyens de protection, par exemple des soufflets de protection, visant à protéger la tige de l'actionneur 7.

Une fois terminé l'extraction, l'embout d'appui 15 peut être amovible dans l'outil d'appui 6 de sorte que l'utilisateur peut changer l'embout d'appui 15 sans détacher l'outil d'appui 6 des glissières..

## Revendications

1. Un appareil d'injection (1), avantageusement destiné à l'injection sous-corticale d'une substance dans une plante, comprenant :
▪ un corps (2),
▪ une tête d'injection comprenant :
- un porte-aiguille (4) dans lequel est maintenu une aiguille (5) sensiblement rectiligne et définissant l'axe longitudinal (A) de l'appareil, laquelle aiguille (5) est percée en son centre d'un canal débouchant par au moins un trou sur la paroi de l'aiguille (5) et donc pas à l'extrémité de celle-ci destinée à pénétrer dans la plante ; et
- un guide (3) qui présente une cavité dans laquelle le porte-aiguille (4) et l'aiguille (5) peuvent se mouvoir selon l'axe longitudinal (A) de l'appareil (1), laquelle cavité présente une ouverture de sorte à permettre le passage de l'aiguille (5) et son insertion dans l'écorce de la plante ;
▪ un premier actionneur (9) apte à agir sur un réservoir (8) de sorte à mettre en pression la substance stockée dans le réservoir (8) et à permettre l'injection de cette substance au travers de l'aiguille (5) jusqu'à la plante via le canal ;
▪ un outil d'appui (6) de l'appareil (1) sur la plante, lequel outil d'appui (6) est apte à être positionné dans l'axe longitudinal de l'appareil et se trouve à proximité de l'aiguille (5), de préférence cet outil d'appui présente un orifice laissant passer l'aiguille (5),
▪ un deuxième actionneur (7), apte à agir sur l'outil d'appui (6) de sorte à permettre l'extraction de l'aiguille (5) après son insertion dans la plante;
dans lequel
- l'outil d'appui (6) constitue également un manchon protecteur pour l'extrémité de l'aiguille (5),
- le deuxième actionneur (7) est également apte à rétracter l'outil d'appui (6) au fur et à mesure de l'insertion de l'aiguille (5) dans la plante ; et
- l'appareil d'injection (1) comprend en outre un mécanisme de frappe (10), apte à percuter le porte-aiguille (4) avec une puissance inférieure à 10 Joules, de préférence supérieure à 1,0 Joule, de sorte à permettre l'insertion de l'aiguille (5) dans l'écorce de la plante.

2. L'appareil d'injection (1) selon la revendication précédente, **caractérisé en ce que** le mécanisme de frappe est un système de percussion électromécanique ou électropneumatique.

3. L'appareil d'injection (1) selon l'une quelconque des revendications précédentes, **caractérisé en ce que** le porte-aiguille et l'aiguille sont solidaires du corps de l'appareil et présentent, avec le corps de l'appareil (1), un jeu compris entre 2 et 15 mm dans l'axe longitudinal (A) de l'appareil.

4. L'appareil d'injection (1) selon l'une quelconque des revendications précédentes, **caractérisé en ce que** l'outil d'appui comprend un embout d'appui apte à venir en contact contre l'écorce de la plante.

5. L'appareil d'injection (1) selon l'une quelconque des revendications précédentes, **caractérisé en ce qu'**il comprend en outre un premier capteur d'effort (16) apte à mesurer l'effort d'appui de l'outil d'appui (6) contre l'écorce de la plante et au moins un microcontrôleur qui permet :
i) dès lors qu'un effort minimum, correspondant à la valeur d'activation, est détecté, l'activation du deuxième actionneur (7) de sorte à rétracter l'outil d'appui (6) et à permettre l'insertion de l'aiguille (5) dans l'écorce de la plante ;
ii) dès lors que l'effort d'appui est inférieur à une valeur seuil dite valeur de frappe, l'activation du mécanisme de frappe (10) ;
iii) ) dès lors que l'effort d'appui devient inférieur à une valeur limite, une désactivation du deuxième actionneur (7).

6. L'appareil d'injection (1) selon l'une quelconque des revendications précédentes, **caractérisé en ce qu'**il comprend en outre un premier capteur de position, associé au deuxième actionneur (7), apte à mesurer la profondeur d'insertion de l'aiguille (5) dans l'écorce de la plante et au moins un microcontrôleur qui permet, dès lors qu'une profondeur cible est atteinte pour la plante dans laquelle l'aiguille (5) est insérée, une désactivation du deuxième actionneur (7) et du mécanisme de frappe (10) et, de préférence permet l'activation du premier actionneur (9), apte à agir sur le réservoir (8) de sorte à mettre en pression la substance stockée dans celui-ci, et l'injection de la substance au travers de l'aiguille (5) jusqu'à la plante.

7. L'appareil d'injection (1) selon l'une quelconque des revendications précédentes, **caractérisé en ce que** le mécanisme de frappe (10) présente une puissance comprise entre 1,2 et 8 Joules, de préférence entre 1,2 et 4 Joules.

8. L'appareil d'injection (1) selon l'une quelconque des revendications précédentes, **caractérisé en ce que** le guide (3) est monté pivotant, par rapport au corps (2), autour d'un axe transversal (B) perpendiculaire à l'axe longitudinal (A) de l'appareil.

9. L'appareil d'injection (1) selon l'une quelconque des revendications précédentes, **caractérisé en ce que** le porte-aiguille (4) intègre une monture qui peut soit être partie intégrante du porte-aiguille, soit consister en une pièce indépendante que l'on vient fixer sur le porte-aiguille (4).

10. Un procédé de traitement d'une plante par injection sous-corticale d'une substance, lequel procédé comprend les étapes suivantes :
• application, sur l'écorce de la plante, d'un appareil tel que défini à l'une quelconque des revendications 1 à 9,
• activation dudit appareil (1), de sorte à permettre l'insertion de l'aiguille (5), au sein de l'écorce de la plante, puis à permettre l'injection sous-corticale de la substance , et
• extraction de l'aiguille (5) de l'écorce de la plante par l'activation du deuxième actionneur (7) dudit appareil (1).

11. Une utilisation d'un appareil (1) tel que défini à l'une quelconque des revendications 1 à 9 en vue de permettre l'injection d'une substance sous l'écorce d'une plante.

## Patentansprüche

1. Injektionsgerät (1), vorteilhafterweise zur subkortikalen Injektion einer Substanz in eine Pflanze bestimmt, umfassend:
• einen Körper (2),
• einen Injektionskopf, umfassend:
- einen Nadelträger (4), in dem eine im Wesentlichen geradlinige Nadel (5) gehalten wird, die eine Längsachse (A) des Gerätes definiert, wobei diese Nadel (5) in ihrem Zentrum in einem Kanal durchbohrt ist, der in mindestens einem Loch auf der Wand der Nadel (5) ausmündet und also nicht an ihrem Ende, das dazu bestimmt ist, in die Pflanze einzudringen, und
- eine Führung (3), die einen Hohlraum aufweist, in dem sich der Nadelträger (4) und die Nadel (5) in der Richtung der Längsachse (A) des Gerätes (1) bewegen können, wobei der genannte Hohlraum eine Öffnung aufweist, derart, dass der Durchtritt der Nadel (5) und ihr Einführen in die Rinde der Pflanze ermöglicht wird,
• ein erstes Betätigungsorgan (9), geeignet, derart auf ein Reservoir (8) zu wirken, dass die im Reservoir (8) gelagerte Substanz unter Druck gesetzt wird und die Injektion dieser Substanz durch die Nadel (5) über den Kanal bis in die Pflanze ermöglicht wird,
• ein Werkzeug (6) zum Aufsetzen des Gerätes (1) auf die Pflanze, wobei dieses Aufsetzwerkzeug (6) dazu geeignet ist, in der Längsachse des Gerätes angeordnet zu werden und sich nahe der Nadel (5) befindet, wobei dieses Aufsetzwerkzeug vorzugsweise eine Öffnung aufweist, die die Nadel (5) durchtreten lässt,
• ein zweites Betätigungsorgan (7), geeignet, auf das Aufsetzwerkzeug (6) zu wirken, um das Herausziehen der Nadel (5) nach ihrer Einführung in die Pflanze zu erlauben,
in dem
- das Aufsetzwerkzeug (6) auch eine Schutzhülse für das Ende der Nadel (5) darstellt,
- das zweites Betätigungsorgan (7) auch geeignet ist, das Aufsetzwerkzeug (6) nach und nach während der Einführung der Nadel (5) in die Pflanze zurückzuziehen, und
- das Injektionsgerät (1) außerdem einen Schlagmechanismus (10) umfasst, der geeignet ist, auf den Nadelträger (4) mit einer ***Energie geringer als 10 Joules und vorzugsweise größer als 1,0 Joule zu schlagen, um die Einführung der Nadel (5) in die Rinde der Pflanze zu erlauben.

2. Injektionsgerät (1) nach dem vorangehenden Patentanspruch, **dadurch gekennzeichnet, dass** der Schlagmechanismus ein elektromechanisches oder elektropneumatisches Aufschlagsystem ist.

3. Injektionsgerät (1) nach irgendeinem der vorangehenden Patentansprüche, **dadurch gekennzeichnet, dass** der Nadelträger und die Nadel mit dem Körper des Gerätes fest verbunden sind und mit dem Körper des Gerätes (1) ein Spiel von zwischen 2 und 15 mm in der Längsrichtungsachse (A) des Gerätes aufweisen.

4. Injektionsgerät (1) nach irgendeinem der vorangehenden Patentansprüche, **dadurch gekennzeichnet, dass** das Aufsetzwerkzeug ein Aufsetzendstück aufweist, das geeignet ist, mit der Rinde der Pflanze in Berührung zu kommen.

5. Injektionsgerät (1) nach irgendeinem der vorangehenden Patentansprüche, **dadurch gekennzeichnet, dass** es außerdem einen ersten Kraftsensor (16) umfasst, der geeignet ist, die Andruckkraft des Gerätes (6) an die Rinde der Pflanze zu messen und mindestens eine Mikrosteuerkomponente, die erlaubt:
i) die Betätigung des zweiten Betätigungsorgans (7) erlaubt, sobald eine Minimalkraft, die einem Aktivierungswert entspricht, festgestellt wird, derart, dass das Aufsetzwerkzeug (6) zurückgezogen wird und die Einführung der Nadel (5) in die Rinde der Pflanze ermöglicht wird,
ii) die Betätigung des Schlagmechanismus (10) erlaubt, wenn die Andruckkraft kleiner ist, als ein Schlagwert genannter Schwellenwert,
iii) eine Deaktivierung des zweiten Betätigungsorgans (7) erlaubt, sobald die Andruckkraft einen Grenzwert unterschreitet.

6. Injektionsgerät (1) nach irgendeinem der vorangehenden Patentansprüche, **dadurch gekennzeichnet, dass** es außerdem einen ersten Positionssensor in Verbindung mit dem zweiten Betätigungsorgans (7) aufweist, der geeignet ist, die Einführtiefe der Nadel (5) in die Rinde der Pflanze zu messen und mindestens eine Mikrosteuerkomponente, die eine Deaktivierung des zweiten Betätigungsorgans (7) und des Schlagmechanismus (10) erlaubt, sobald eine Zieltiefe für die Pflanze, in die die Nadel (5) eingeführt wird, erreicht ist, und vorzugsweise die Betätigung des ersten Betätigungsorgans (9) erlaubt, das geeignet ist, auf das Reservoir (8) derart zu wirken, dass die in diesem gelagerte Substanz unter Druck gesetzt wird, und die Injektion der Substanz durch die Nadel (5) bis in die Pflanze erlaubt.

7. Injektionsgerät (1) nach irgendeinem der vorangehenden Patentansprüche, **dadurch gekennzeichnet, dass** der Schlagmechanismus (10) eine Leistung zwischen 1,2 und 8 Joules aufweist, vorzugsweise zwischen 1,2 und 4 Joules.

8. Injektionsgerät (1) nach irgendeinem der vorangehenden Patentansprüche, **dadurch gekennzeichnet, dass** die Führung (3) relativ zum Körper (2) um eine Querachse (B) kippbar montiert ist, die auf der Längsachse (A) des Gerätes senkrecht steht.

9. Injektionsgerät (1) nach irgendeinem der vorangehenden Patentansprüche, **dadurch gekennzeichnet, dass** der Nadelträger (4) eine Fassung umfasst, die entweder einstückiger Bestandteil des Nadelträgers ist, oder ein unabhängiges Stück darstellt, das auf dem Nadelträger (4) befestigt wird.

10. Verfahren zur Behandlung einer Pflanze durch subkortikale Injektion einer Substanz, das die folgenden Schritte umfasst:
• Aufsetzen eines Gerätes nach irgendeinem der Patentansprüche 1 bis 9 auf die Rinde der Pflanze,
• Betätigung des genannten Gerätes (1), um das Einführen der Nadel (5) in die Rinde der Pflanze zu ermöglichen und dann die subkortikale Injektion der Substanz zu ermöglichen, und
• Herausziehen der Nadel (5) aus der Rinde der Pflanze durch Betätigung des zweiten Betätigungsorgans (7) des genannten Gerätes (1)

11. Gebrauch eines Gerätes (1) nach irgendeinem der Patentansprüche 1 bis 9 zur Injektion einer Substanz unter die Rinde einer Pflanze.

## Claims

1. An injection device (1), preferably intended for the subcortical injection of a substance into a plant, comprising:
▪ A body (2).
▪ An injector head comprising:
- A needle holder (4) in which a substantially straight needle (5) is held and defines the longitudinal axis (A) of the device, said needle (5) is pierced in its center by a channel opening through at least one hole on the side wall of the needle (5), and therefore not at the end thereof intended to penetrate the plant;
- A guide (3) which has a cavity in which the needle holder (4) and the needle (5) can move along the longitudinal axis (A) of the device (1), which cavity has an opening so as to allow the passage of the needle (5) and its insertion into the bark of the plant;
▪ A first actuator (9) capable of acting on a tank (8) so as to pressurize the substance stored in the tank (8) and to allow the injection of this substance through the needle (5) to the plant via the channel,
▪ a support tool (6) from the device (1) on the plant, which support tool (6) is able to be positioned in the longitudinal axis of the device and is located close to the needle (5), preferably this support tool has an orifice allowing the needle (5) to pass through,
▪ a second actuator (7), able to act on the support tool (6) so as to allow the extraction of the needle (5) after its insertion into the plant
wherein:
- The support tool (6) also acts as a protective sleeve for the end of the needle (5);
- The second actuator (7) is also capable of retracting the support tool (6) as the needle (5) is inserted into the plant; and
- The injection device (1) also includes a striking mechanism (10) capable of striking the needle holder (4), with a power of less than 10 Joules, preferably greater than 1.0 Joule, so as to allow the insertion of the needle (5) into the plant.

2. The injection device (1) according to the preceding claim, **characterized in that** the striking mechanism (10) is an electromechanical or electropneumatic system.

3. The injection device (1) according to any one of the preceding claims, **characterized in that** the needle holder (4) and the needle (5) are integral with the body (2) of the device and have, with it, a clearance of between 2 and 15 mm along the longitudinal axis (A) of the device (1).

4. The injection device (1) according to any one of the preceding claims, **characterized in that** the support tool (6) comprises a support jack (15) capable of coming into contact with the bark of the plant.

5. The injection device (1) according to any one of the preceding claims, **characterized in that** it further comprises a first force sensor (16) capable of measuring the applied force of the support tool (6) against the bark of the plant and at least one microcontroller which enables:
i) as soon as a minimum effort, corresponding to the activation value, is detected, the activation of the second actuator (7) so as to retract the support tool (6) and allow the insertion of the needle (5) into the bark of the plant;
ii) as soon as the applied force is less than a threshold value called the strike value, the activation of the strike mechanism (10);
iii) as soon as the applied force becomes less than a limit value, deactivation of the second actuator (7).

6. The injection device (1) according to any one of the preceding claims, **characterized in that** it further comprises a first position sensor, associated with the second actuator (7), capable of measuring the depth of insertion of the needle (5) into the bark of the plant, and at least one microcontroller allowing, once the target depth is reached for the plant into which the needle (5) is inserted, a deactivation of the second actuator (7) and the striking mechanism (10) and, preferably the activation of the first actuator (9) capable of acting on the tank (8) so as to pressurize the substance stored therein, and the injection of the substance through the needle (5) up to the plant.

7. The injection device (1) according to any one of the preceding claims, **characterized in that** the striking mechanism (10) has a power of between 1.2 and 8 Joules, preferably between 1.2 and 4 Joules.

8. The injection apparatus (1) according to any one of the preceding claims, **characterized in that** the guide (3) is pivotally mounted, relative to the body (2), around a transverse axis (B) perpendicular to the longitudinal axis (A) of the device.

9. The injection device (1) according to any one of the preceding claims, **characterized in that** the needle holder comprises a frame can either be an integral part of the needle holder (4) or consist of an independent part which is fixed to the needle holder (4).

10. A method for treating a plant by subcortical injection of a substance; said method comprising the following steps:
• application of a device (1), as defined in any one of claims 1 to 9, to the bark of the plant,
• activation of said device (1) so as to allow the insertion of the needle (5) under the bark of the plant and the subsequent subcortical injection of the substance, and
• extraction of the needle (5) from the plant by activation of the second actuator (7) of said device (1).

11. The use of a device (1) as defined in any one of claims 1 to 9 for allowing the injection of a substance under the bark of a plant.
